Europäisches Patentamt

European Patent Office

Office européen des brevets

(11) Publication number: **0 380 451**
**A2**

## EUROPEAN PATENT APPLICATION

(21) Application number: **90830008.0**

(22) Date of filing: **08.01.90**

(51) Int. Cl.5: **A61B 17/56, A61F 2/00**

(30) Priority: **11.01.89 IT 1906689**

(43) Date of publication of application:
**01.08.90 Bulletin 90/31**

(84) Designated Contracting States:
**AT BE CH DE ES FR GB GR LI NL SE**

(71) Applicant: **G. CREMASCOLI S.p.A.**
**Via Clemente Prudenzio, 14/16**
**I-20138 Milano(IT)**

(72) Inventor: **Ghisellini, Franco, G. Cremascoli**
**S.p.A.**
**Via Clemente Prudenzio, 14/16**
**I-20138 Milano(IT)**

(74) Representative: **Cicogna, Franco**
**Ufficio Internazionale Brevetti Dott.Prof.**
**Franco Cicogna Via Visconti di Modrone,**
**14/A**
**I-20122 Milano(IT)**

(54) **Apparatus for resecting femurs and applying knee articulation prostheses.**

(57) The present invention relates to an apparatus for properly resecting femurs and applying knee articulation prostheses, which apparatus comprises a main body (1) including a fixed body (2) and a movable body (3) which can be coupled to one another.

With the main body (1) there are removably associated a bottom bracket (23) and a locating body (32), adapted to define guides for carrying out front and rear femur resections.

A top body (50) is moreover provided, which can be removably coupled to the main body (1) in order to carry out the transversal resection and the tapering resection of the distal portion of the femur bone.

Fig.1

EP 0 380 451 A2

## BACKGROUND OF THE INVENTION

The present invention relates to an apparatus for properly resecting femurs and applying knee articulation prostheses.

As is known,in the case of malfunctions of the knee articulations, as due to fractures, arthritis,tumoural diseases and the like, it is frequently necessary to use replacement prostheses for this articulation, either of the complete or of the partial type.

In order to apply these prostheses, the femur head must be resected.

For resecting the femur head, resecting apparatus are already known which must be located at the femur head so as to provide locating and guide points for the resection operation.

On the other hand, known femur head resection apparatus have several drawbacks, the most important of which is that these apparatus can be applied only with great diffculties and frequently do not afford the possibility of precisely resecting the femur head.

## SUMMARY OF THE INVENTION

Accordingly, the aim of the present invention is to overcome the above mentioned drawbacks by providing an apparatus for properly resecting femurs and applying knee articulation prostheses, which apparatus can be easily and quickly fitted to different morphologies of the femur bone.

Within the scope of the above mentioned aim, a main object of the present invention is to provide such a femur resection apparatus which can be perfectly fitted to the slant of the diaphysisfemur axis of the patient with respect to a vertical axis.

Another object of the present invention is to provide such a femur resection apparatus which is very reliable and safe in operation.

Yet another object of the present invention is to provide such a femur head resection apparatus which is very simple construction-wise.

According to one aspect of the present invention, the above mentioned aim and objects, as well as yet other objects, which will become more apparent hereinafter, are achieved by an apparatus for properly resecting femurs and applying knee articulation prostheses, characterized in that said apparatus comprises a main body including a fixed body and a movable body which can be coupled to one another.

With the mentioned main body there are removably associated a bottom bracket and a locating body, adapted to define guides for carrying out front and rear femur resection operations, there being moreover provided a top body which can be removably coupled to said main body in order to transversely resect the distal portion of the femur bone.

## BRIEF DESCRIPTION OF THE DRAWINGS

Furhter characteristics and advantages of the invention will become more apparent from the following detailed disclosure of a preferred, though not exclusive,embodiment of an apparatus for properly resecting femurs and applying knee articulation prostheses, which is illustrated, by way of an indicative, but not limitative, example in the accompanying drawings, in which:

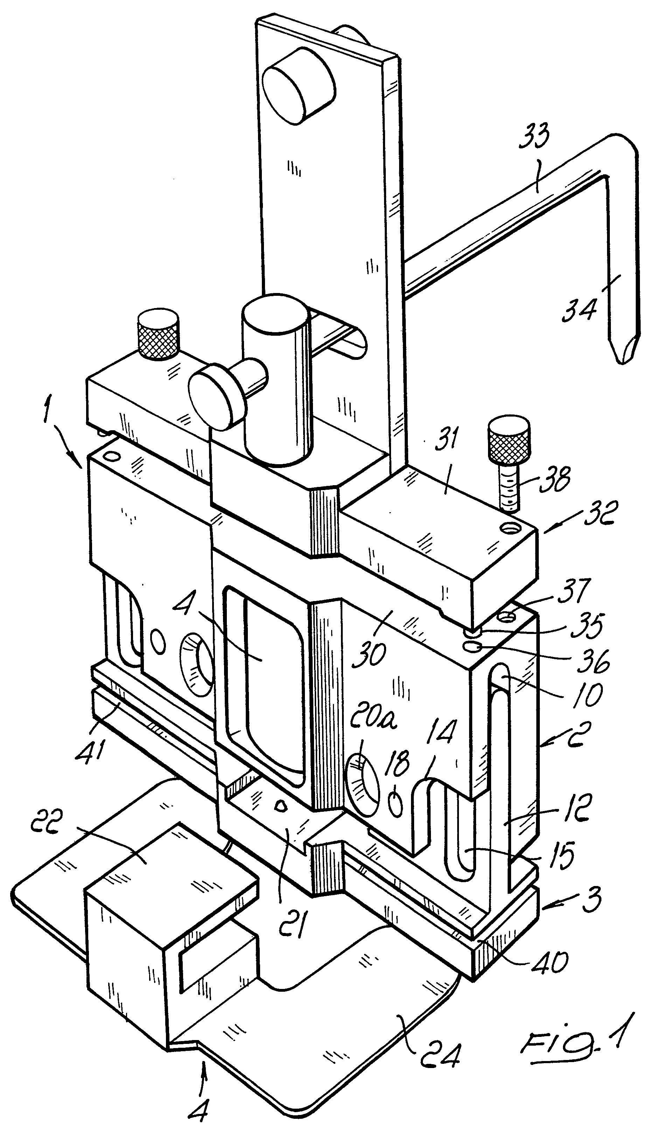

Figure 1 is a schematic perspective exploded view showing the femur resection apparatus according to the present invention;

Figure 2 is a front view of the subject apparatus including the bracket and locating body;

Figure 3 is a side elevation view of the subject apparatus;

Figure 4 is a bottom plan view of the top body included in the apparatus;

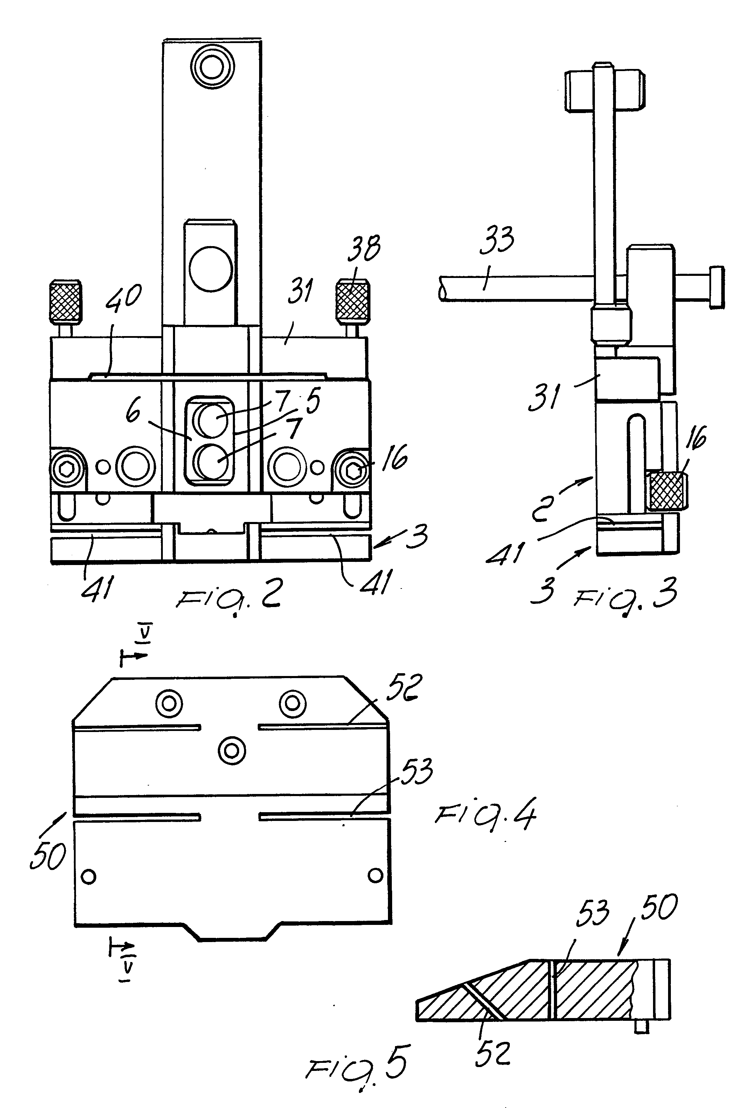

Figure 5 is a cross-sectional view taken along the line V-V of Figure 4;

Figure 6 is a front elevation view of the fixed body;

Figure 7 is a side elevation view of the same fixed body;

Figure 8 is a cross-sectional view taken along the section line VIII-VIII of Figure 6;

Figure 9 is a cross-sectional view taken along the section line IX-IX of Figure 6;

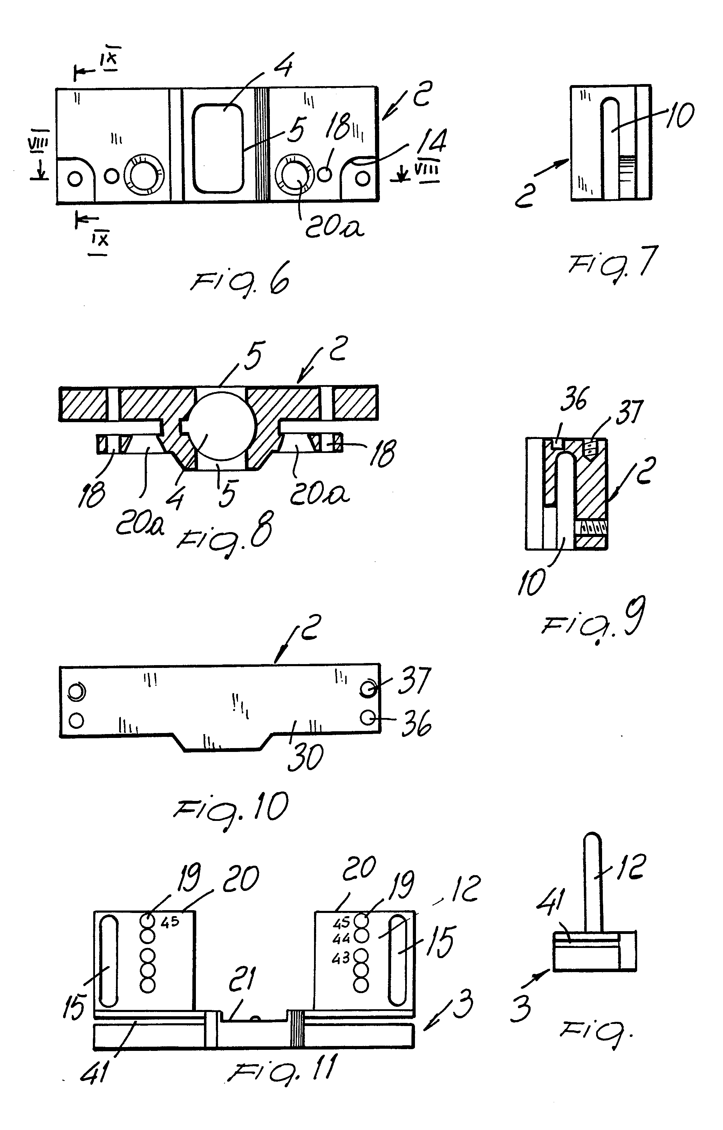

Figure 10 is a top plan view of the fixed body;

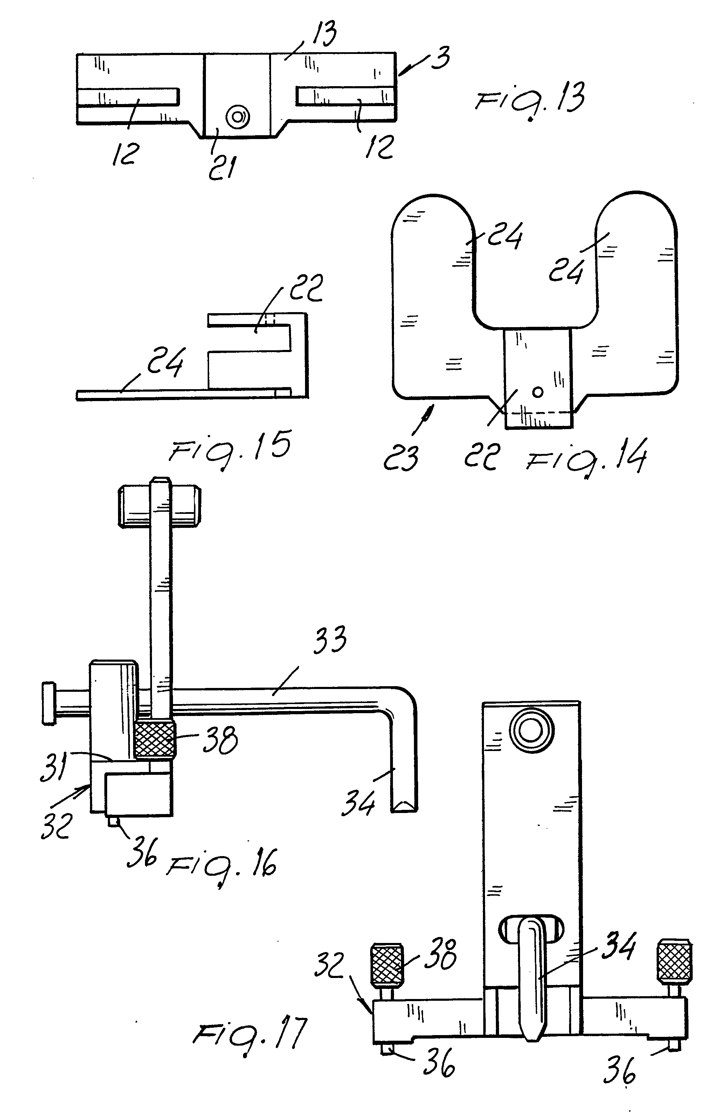

Figure 11 is a front elevation view of the movable body;

Figure 12 is a side elevation view of the movable body;

Figure 13 is a top plan view of the movable body;

Figure 14 is a top plan view of the bracket;

Figure 15 is a side elevation view of the bracket;

Figure 16 is a side elevation view of the locating body;

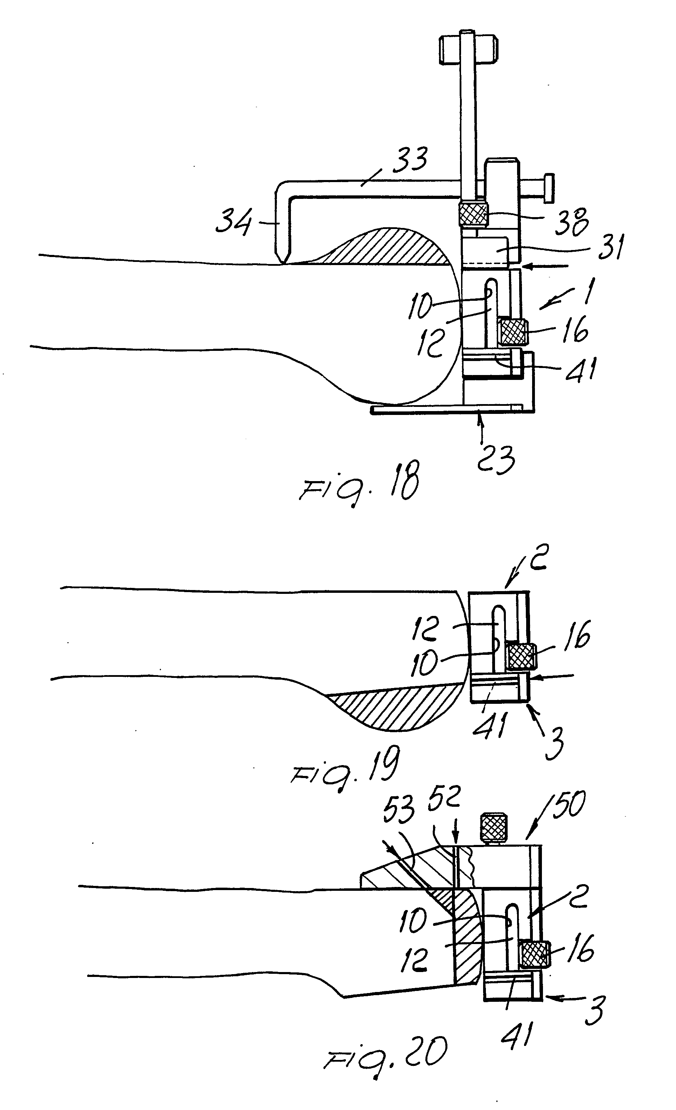

Figure 17 is a front elevation view of the locating body;

Figure 18 is a schematicview showing the subject apparatus applied to the femur bone during a front resection operation;

Figure 19 shows the subject apparatus during the rear resection operation;

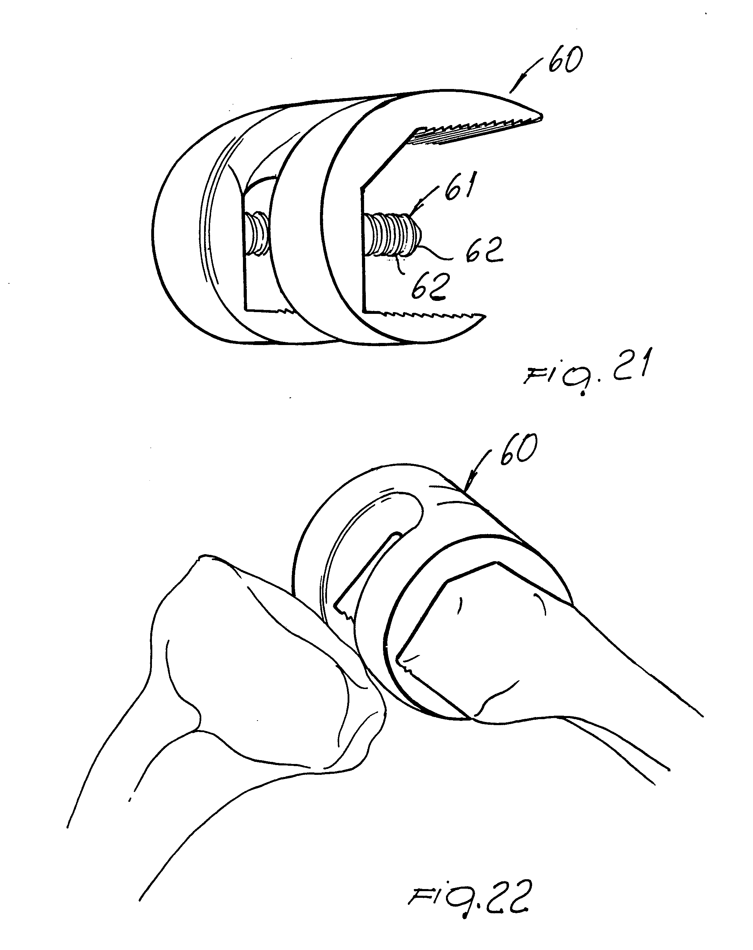

Figure 20 is another schematic view showing the apparatus during the transverse resection operation and tapering resection operation;

Figure 21 is a schematic perspective view showing a femur prosthesis; and

Figure 22 schematically shows the femur prosthesis applied to the femur bone.

## DESCRIPTION OF THE PREFERRED EMBODIMENT

With reference to the figures of the accompanying drawings, the apparatus for properly resecting femur and applying knee articulation prostheses according to the invention comprises a main body, indicated overally at the reference number 1, which has substantially an elongated parallelepipedal shape.

More specifically, the main body 1 comprises a fixed body 2 and a movable body, indicated overally at the reference number 3.

The body 2 is provided, at the central portion thereof, with a cylindrical hollow 4, open at the bottom thereof, which comprises a pair of opposite windows 5 formed on the main surfaces of the fixed body 2.

In this hollow 4 a cylindricalbush 6 can be housed, said cylindrical bush being provided with a longitudinal rib which prevents said cylindrical bush from rotating within the hollow 4.

The cylindrical bush 6 comprises a pair of cross holes 7, arranged with a predetermined slant, and which, owing to the reversable position of the cylindrical bush 6, afford the possibility of defining the hole for both the right and left articulation.

The fixed body 2 is provided, at a longitudinal middle portion thereof, with a cut 10 in which can be slidingly arranged the leg 12 projecting from the base 13 of the movable body 3.

The fixed body 2 is provided, at the front thereof, with cut-outs 14 arranged at longitudinal slots 15 defined by the leg 12 of the movable body 3, so as to afford the possibility of applying a locking screw 16 which can be thread engaged in a corresponding threaded seat defined in the fixed body 2 at the registering wall of the cut-outs 14, so as to lock at a set position the body 3 with respect to the fixed body 2.

Moreover, the front wall of the body 2 is provided with throughgoing holes 18 therein can be engaged pins to be inserted into the femur bone and passing through a plurality of holes 19 correspondingly formed through the leg and associated with number references, indicated at 20, which can be registered with tapering holes 20a formed on the front wall of the fixed body 2.

At its central bottom portion, the movable body 3 is provided with a recess 21, therein can be engaged a jaw 22, defined by a bracket body 23 provided with abutment lugs 24 which will be disclosed in a more detailed way hereinafter.

At the top, the fixed body 2 is provided with an abutment surface 30, therewith can be associated the base plate 31 of a locating body, indicated overally at the reference number 32 and provided with an abutment lug 33 including a right angle bent end portion 24.

Moreover, from the base plate 31, extend centering pins 35 which can be engaged in corresponding centering seats 36 formed by the abutment surface 30.

In order to affix the prosthesis there is moreover provided a threaded seat 37 therein can be thread engaged a screw 38 passing through the plate 31.

The base plate 31, applied to the fixed body 2, is provided with a slot 40, which constitutes the guide seat for carrying out the front resection operation, whereas the movable body is provided at the bottom thereof with a bottom guide slot 41 in order to carry out the rear resection operation.

The above mentioned locating body 31 is removable and can be replaced by a top body 50 also including centering pins 51 which can be engaged in the centering seats 36.

The top body 50 is moreover provided with a cross cut 52 which operates as a guide element for the transverse resection operation, as well as with a slant cut 53 for carrying out the tapering resection operation.

In order to properly use the femur resection apparatus according to the present invention, a hole is made at the start at the centre of the infracondyloid groove by using the holes 7 of the cylindrical bush 6.

Then, by using an endomidullar femoral nail, to be inserted into the throughgoing holes 18, the main body 1 is brought into contact with the femur head.

Then, the locating body 32 is affixed, so as to fit the biasing action provided by the end of the lug 33 on the femur neck.

After having carried out this operation, the main body 1 is further restrained to the femur head, by means of the bracket 23, and the movable body is locked with respect to the fixed body.

After having carried out this preliminary operations, the front portion and rear portion of the femur are resected, as is schematically shown in figures 18 and 19.

Then, after having removed the locating body 32, the top body 50 is applied and, through the slots 52 and 53, are carried out the cross resection of the femur condyles and the tapering resection.

After having preset the head of the femur bone, there is applied the prosthesis, indicated overall at the reference number 60, by locking it in the femur head, by inserting by force the pins 61 provided with annular ridges 62.

From the above disclosure it should be apparent that the invention fully achieves the intended aim and objects.

In particular, it is to be pointed out that a femur head resection apparatus has been provided which affords the possibility of resecting the femur head in a precise and quick way.

While the invention has been disclosed and illustrated with reference to a preferred embodiment thereof, it should be apparent that the disclosed embodiment is susceptible to several modifications and variations all of which will come within the scope and spirit of the appended claims.

**Claims**

1. An apparatus for properly resecting femurs and applying knee articulation prostheses, characterized in that said apparatus comprises a main body, including a fixed body and a movable body coupled to one another, with said main body there being removably associated a bottom bracket and a locating body, to define guides for carrying out front and rear femur resection operations, there being moreover provided a top body which can be removably coupled to said main body to carry out the transverse resection operation of the distal portion of the femur bone.

2. An apparatus according to claim 1, characterized in that said fixed body is provided, on a middle portion thereof, with a substantially cylindrical hollow having opening windows provided on the main surfaces of said fixed body which has a substantially flat parallelepipedal configuration.

3. An apparatus according to the preceding claims, characterized in that in said hollow there is housed a cylindrical bush including a longitudinal rib for preventing said bush from rotating in said hollow, said cylindrical bush further comprising a pair of cross holes having a set slant arrangement.

4. An apparatus according to one or more of the preceding claims, characterized in that said fixed body is provided, at a middle portion thereof, with a cut therein can be removably slidingly engaged a leg extending from said movable body.

5. An apparatus according to one or more of the preceding claims, characterized in that said fixed body is provided, at the front portion thereof, with cut-outs arranged at elongated slots formed on said leg, said fixed body being moreover provided with a threaded seat therein engages a screw for locking said leg with respect to said fixed body.

6. An apparatus according to one or more of the preceding claims, characterized in that said fixed body further comprises throughgoing holes therein there are engaged pins to be inserted into one of the holes formed on said leg.

7. An apparatus according to one or more of the preceding claims, characterized in that the holes of said legs are referenced by reference marks which can be arranged at tapering and registering holes formed on said fixed body.

8. An apparatus according to one or more of the preceding claims, characterized in that said movable body is provided, at the bottom thereof, with a seat to be engaged by a jaw for affixing said bracket.

9. An apparatus according to one or more of the preceding claims, characterized in that said fixed body is provided, at the top thereof, with an abutment surface thereon can abut a base plate of said locating body, said base plate being provided with centering pins adapted to be engaged in corresponding seats, there being moreover provided threaded seats to be engaged by locking screw means, and said locating body being provided with a right angle bent lug for properly arranging said locating body with respect to the neck of the femur.

10. An apparatus according to one or more of the preceding claims, characterized in that said locating body defines, in cooperation with said abutment surface, a top guide slot for the front femur resection operations,whereas the movable body defines top guide slots for the rear femur resection operation.

11. An apparatus according to one or more of the preceding claims, characterized in that said top body is provided with centering pins which can be engaged in the centering seats of the abutment surface, said top body being provided with a cross slot for the transverse resection operation and a slant slot for the tapering resection operation.

12. An apparatus according to one or more of the preceding claims, characterized in that said apparatus comprises a femur prosthesis adapted to be coupled on the head of a femur bone, to be resected at the front and rear thereof, and arranged for the transverse and tapering resection, said prosthesis being provided with a fixing pin including locking annular ridges.

Fig.1

FIG. 2

FIG. 3

FIG. 4

FIG. 5

Fig. 6

Fig. 7

Fig. 8

Fig. 9

Fig. 10

Fig. 11

Fig.

*Fig.13*

*Fig.15*

*Fig.14*

*Fig.16*

*Fig.17*

Fig. 18

Fig. 19

Fig. 20

Fig. 21

Fig. 22